# EUROPEAN PATENT APPLICATION

(11) **EP 4 575 506 A1**
(43) Date of publication of application: **25.06.2025**
(21) Application number: 23218045.5
(22) Date of filing: 19.12.2023
(51) Int. Cl.: G01N 35/00

(54) **CALIBRATION MANAGEMENT OF AN IN-VITRO DIAGNOSTIC SYSTEM**

(71) Applicant: F. Hoffmann-La Roche AG, 4070 Basel (CH); Roche Diagnostics GmbH, 68305 Mannheim (DE)
(72) Inventor: Bruckner, Richard, 6343 Rotkreuz (CH); Eicher, Andreas, 6343 Rotkreuz (CH); Gan, Li, 6343 Rotkreuz (CH); Schwesig-Stelzer, Franz Korbinian, 6343 Rotkreuz (CH)
(74) Representative: Curcio, Mario

(57) **Abstract**

A computer-implemented method of automatically managing calibration of an in-vitro diagnostic (IVD) system comprising executing calibration cycles/procedures comprising using calibration solutions, using a calibration solution comprising a first step of transporting the calibration solution to a measurement unit and a second step of measuring the calibration solution by the measurement unit. For each calibration cycle the method comprises starting repetition of the calibration procedure at the earliest at a start of a buffer time period before expiry of a respective calibration validity period, interrupting the calibration procedure upon receiving an order to execute a sample IVD test and restarting or resuming the calibration procedure after executing the sample IVD test, as long as repetition of the calibration procedure can be restarted or resumed within the buffer time period. Interrupting the calibration procedure comprises different steps depending on whether the order to execute a sample IVD test is received during using a first calibration solution or a subsequent calibration solution in a series of calibration solutions and whether it is received during the first step of transporting the first or a subsequent calibration solution to the measurement unit or the second step of measuring the first or a subsequent calibration solution. An IVD system performing operations associated with the method of automatically managing calibration is also disclosed.

## Description

### Field of the invention

The present disclosure relates to a computer-implemented method of automatically managing calibration of an in-vitro diagnostic system and to an in-vitro diagnostic system performing operations associated with the method of automatically managing calibration.

### Background

In medicine, doctor's diagnosis and patient treatment often relies on the measurement of the concentration of analytes or other parameters in a patient sample by carrying out in-vitro diagnostic (IVD) tests. These measurements are typically carried out by in-vitro diagnostic systems that can be configured to analyze certain types of samples and detect certain types of analytes using various detecting technologies. As the life of patients may depend on the precision and the reliability of such measurements it is important that the systems perform correctly.

It is a general requirement for in-vitro diagnostic systems to implement a set of Quality Control (QC) procedures in order to check that they continue operating correctly.

One of these procedures is calibration. In most cases calibration is performed using standard solutions, with known concentrations. In this way it is possible to correlate a measured signal to a quantitative result. Calibration should be performed more or less frequently depending on the system and other variable factors which may affect performance. One of these factors can be aging of reagents and/or other solutions used, leading to expiration of reagents and/or other solutions a predetermined time after they have been exposed to environmental conditions. The signal stability of a measurement unit, especially for some types of measurement units, including e.g. biosensors, can be another factor. In particular, some detectors and sensors used to measure analyte concentrations can be subject to interferences, e.g. due to presence and/or high concentration of interfering substances in a particular test sample, and can be subject to at least temporary signal instability. In particular, some sensors may experience a signal drift that in some cases may remain unnoticed. This can lead to measurement errors and therefore may require more frequent calibrations as well as so called QC measurements between consecutive calibrations, by measuring one or more reference samples, also called QC samples, with known values of the analytes or parameters of interest, in the same way as test samples are measured, in order to further check that the calibrated instrument is actually within the specifications or admissible range. Thus, in general, there is a time limit for the validity of a calibration.

It is typically the case that during a calibration procedure an analyzer or component thereof is not available for use, e.g. not available to accept new samples. Thus, the effective throughput and usability as well as the costs of running an in-vitro diagnostic system may be affected by the fact that a significant time may have to be dedicated to the execution and repetition of calibration procedures. Moreover, an operator may be in the situation to have to wait for the analyzer to become available again, e.g. until a calibration procedure is completed, before being able to input a new sample into the analyzer, whereas, especially in point-of-care environments, e.g. in emergency situations, intensive care units and the like, obtaining quick results that are also reliable can be of utmost importance and any delay can be life critical. Also, for some IVD tests, including determining blood-gas parameters like pO₂ for example, it is important that samples are processed soon after a blood sample is drawn from a patient otherwise the reliability of the IVD test will be affected as a result of exposure to ambient conditions. In addition, operator's time may be wasted, that could be otherwise used more efficiently. If the operator cannot afford waiting because of other more urgent ongoing activities, he or she will typically make a second attempt at a later time, whereas the analyzer had likely become available in the meantime much earlier, thus unnecessarily delaying sample analysis.

### General description

A computer-implemented method of automatically managing calibration of an in-vitro diagnostic (IVD) system and an IVD system comprising a calibration management system running a computer-readable program provided with instructions to perform operations associated with the method of managing calibration that ensure analytical performance of the IVD system, i.e. reliability of the IVD test results, while ensuring availability of the IVD system at all times in order to process samples and to provide IVD test results as quick as possible. Another advantage is gained efficiency in the laboratory workflow by preventing that operator's time is unnecessarily wasted and/or by preventing additional stress in an already highly demanding situation by e.g. having to look for another available IVD system or having to wait until the IVD system becomes available.

In particular, the method comprises executing calibration cycles, a calibration cycle comprising executing a calibration procedure in order to determine test-specific calibration parameters used in evaluating sample IVD test results, and repeating the calibration procedure in order to update the test-specific calibration parameters before expiry of a respective calibration validity period, where different calibrations procedures/cycles possibly have different calibration validity periods respectively, and where executing different calibration procedures/cycles comprises using different series of calibration solutions respectively, a series comprising at least two different calibration solutions, and wherein using a calibration solution comprises a first step of transporting the calibration solution to a measurement unit and a second step of measuring the calibration solution by the measurement unit. For each calibration cycle the method further comprises starting repetition of the calibration procedure at the earliest at a start of a buffer time period before expiry of the respective calibration validity period, interrupting the calibration procedure upon receiving an order to execute a sample IVD test and restarting or resuming the calibration procedure after executing the sample IVD test, as long as repetition of the calibration procedure can be restarted or resumed within the buffer time period. In particular, interrupting the calibration procedure comprises different steps depending on whether the order to execute a sample IVD test is received during using a first calibration solution or a subsequent calibration solution in a series of calibration solutions and whether it is received during the first step of transporting the first or a subsequent calibration solution to the measurement unit or the second step of measuring the first or a subsequent calibration solution.

The term "calibration procedure" as used herein refers to a process of checking if an IVD system is working accurately by comparing known standards with measurement results delivered by the IVD systems. This enables determining a valid relation between the measured value and the actual concentration of an analyte in a sample under actual measurement conditions. Depending on the type of signal and especially linearity or non-linearity of the signal at different concentrations, which may vary depending on the particular sample IVD test, e.g. on the particular sample, on the particular analyte(s) of interest in the sample, on the particular workflow and measuring conditions, the calibration procedure may comprise measuring one or more levels of calibrators corresponding to different concentration ranges of calibration materials or standards that fall in the range of detection of the IVD system (dynamic range) and/or the typical range of concentrations of analytes that can be found in a sample. When only one calibrator level is measured, the calibration procedure is a one-point calibration procedure. When two levels of calibrator are measured, the calibration procedure is a two-point calibration procedure and so on. A "multi-point calibration procedure" is a calibration procedure that comprises measuring a plurality of calibrator levels, i.e., at least two and typically three or more, and in particular measuring a respective calibrator level for each of a plurality of calibration points, thereby obtaining a plurality of respective calibration points.

In accordance with other embodiments, a "calibration procedure" can be also a procedure that enables to correlate a measured sample signal to a qualitative result, i.e., to the mere presence or absence of an analyte. In such case usually criteria are defined such as a threshold or cut-off that separate e.g. normal healthy samples from abnormal pathogenic samples. For qualitative calibration often two calibrators are used: one with no analyte present (negative calibrator) and one with detectable amounts of analyte (positive calibrator).

Depending on the particular sample IVD tests, different calibration procedures may have to be executed, each possibly including a different calibrator or calibrators and eventually different levels of calibrators and/or a different number of levels.

A calibration procedure may include calculating a calibration result that is the process of constructing a line or a curve, or a mathematical function, that has the best fit to the measured calibration points and including a regression analysis that takes into account statistical inference such as how much uncertainty is present in the constructed line or curve because of measurement errors due to unknown and/or random errors occurred in the process, by calculating the amount of variation or dispersion of the measured calibration points (standard deviation). The process may comprise comparing the constructed line or curve to a reference line or curve or previously constructed lines or curves respectively under the same conditions and/or comparing individual calibration points to reference values or previously measured values. In particular, the calibration procedure includes determining test-specific calibration parameters used in evaluating sample IVD test results. Test-specific calibration parameters can be for example a slope of a calibration line or curve, a degree of linearity, a zero point, an offset, an inflection point and the like.

A "calibrator" is a calibration solution that contains known values of one or more calibration materials or standards used for calibration and that is measured under the same conditions as a sample. Calibrators can be provided in different levels that correspond to different concentration ranges of the calibration materials, including zero concentration, i.e., a blank solution. Typically, one or two levels of the same calibrator are used for a one-point or two-point calibration respectively, in case of linear response to analyte concentrations. Three or more calibrator levels, e.g., up to five, six or more levels may be used if the calibration curve is non-linear.

A "calibration material" can be an analyte identical to an analyte of interest, the concentration or value of which is known, or that generates by reaction or derivatization, e.g., by fragmentation, an analyte identical to an analyte of interest, the concentration or value of which is known, or it can be any other equivalent substance or standard, which mimics the analyte of interest or that can be otherwise correlated to a certain analyte of interest or sample parameter.

A "calibration validity period" is a time window having a predefined time length starting from the time when a calibration procedure has been successfully completed by determining the required test-specific calibration parameters, within which time window the determined test-specific calibration parameters are considered valid, i.e. applicable for evaluating sample IVD test results. A calibration validity period is therefore set to expire after a predetermined time, which can be as short as or as long as e.g. 1 or 2 hours, 6 hours, 12 hours, 24 hours or longer, and can vary for different calibration procedures respectively, although some different calibration procedures may have calibration validity periods of the same lengths.

In particular, it is important to repeat a calibration procedure before its respective calibration time period expires, in order to update the test-specific calibration parameters before they become invalid and to ensure continued analytical performance of the IVD system and reliability of the IVD test results.

The term "calibration cycle" as used herein refers therefore to the recurrence of a calibration procedure at regular predefined intervals, i.e. with a predefined frequency, aimed at updating the test-specific calibration parameters before the respective calibration validity period expires. In particular, the time and any action or procedure between a successfully completed calibration procedure and a subsequent successfully completed repetition of the same calibration procedure, comprising replacing the previously determined test-specific calibration parameters with newly determined test-specific calibration parameters, is called calibration cycle.

Particularly, for each calibration cycle, the method comprises starting repetition of the calibration procedure at the earliest at a start of a buffer time period before expiry of the respective calibration validity period, interrupting the calibration procedure upon receiving an order to execute a sample IVD test and restarting or resuming the calibration procedure after executing the sample IVD test, as long as repetition of the calibration procedure can be restarted or resumed within the buffer time period.

The term "buffer time period" as used herein refers to a time period within the calibration validity period closest to the end or expiration of the calibration validity period, which allows sufficient flexibility in starting and, if required, interrupting, restarting or resuming repetition of the calibration procedure, with sufficient time to execute at least one sample IVD test during the interruption if an order for a sample IVD test is received after starting the repetition of the calibration procedure. The repetition of the calibration procedure does not necessarily have to be completed before expiration of the calibration validity period as long as it can be restarted or resumed within the buffer time period before expiration of the calibration validity period. The interruption can take place more than once as long as sufficient time to complete the sample IVD test and to restart or resume the repetition of the calibration procedure is still available. In order to avoid continued interruptions, according to an embodiment, the method comprises waiting a predefined time after executing the sample IVD test before restarting or resuming the calibration procedure as long as repetition of the calibration procedure can be restarted or resumed within the buffer time period. This is because it may happen that an operator approaches the IVD system with more than one sample for executing more than one sample IVD test in consecutive order.

A series of calibration solutions used for executing a calibration procedure may comprise two or more calibration solutions which, in a particular order, have to be transported to a measuring unit of the IVD system and have to be measured therein.

In particular, interrupting the calibration procedure comprises different steps depending on whether the order to execute a sample IVD test is received during using a first calibration solution or a subsequent calibration solution in a series of calibration solutions and whether it is received during the first step of transporting the first or a subsequent calibration solution to the measurement unit or the second step of measuring the first or a subsequent calibration solution.

According to an embodiment, a series of calibration solutions comprises a single pair or multiple pairs of calibration solutions, each pair comprising a common calibration solution as first calibration solution and any other different calibration solution as the subsequent calibration solution.

Thus the term "first calibration solution" may refer to the first calibration solution in a series of two or more calibration solutions or to the first calibration solution in each pair of calibration solutions if the series comprises multiple pairs of calibration solutions.

The first calibration solution may be in particular a solution with a predefined level of calibration materials, e.g. with the lowest or zero level of at least some calibration materials and/or with a most suitable level of at least some calibration materials related to test-specific calibration parameters that require a more frequent update and which therefore is measured more frequently than any other calibration solution.

Analogously, the term "subsequent calibration solution" may refer to the second, third, fourth and so on calibration solution in a series of two or more calibration solutions or to the second calibration solution in each pair of calibration solutions if the series comprises multiple pairs of calibration solutions.

According to an embodiment, when the order to execute a sample IVD test is received during the first step of transporting the first calibration solution in a series and/or the first calibration solution in a pair to the measurement unit, interrupting the calibration procedure comprises completing the first step of transporting the first calibration solution to the measurement unit and executing a measurement step of the first calibration solution with a reduced measurement time and aborting any following steps of the calibration procedure in order to execute the sample IVD test.

The term "with a reduced measurement time" as used herein is a relative term meant to indicate that the time spent to measure the first calibration solution is a predefined time that is shorter than the regular time spent measuring the same calibration solution when the calibration procedure is not interrupted, e.g. about half the time or less.

According to an embodiment, when the order to execute a sample IVD test is received during the second step of measuring the first calibration solution in a series and/or the first calibration solution in a pair, interrupting the calibration procedure comprises completing the second step of measuring the first calibration solution and aborting any following steps of the calibration procedure in order to execute the sample IVD test.

According to an embodiment, when the order to execute a sample IVD test is received during the first step of transporting a subsequent calibration solution in a series and/or the subsequent calibration solution in a pair to the measurement unit, interrupting the calibration procedure comprises aborting the first step of transporting the subsequent calibration solution to the measurement unit and any following steps of the calibration procedure in order to execute the sample IVD test.

According to an embodiment, when the order to execute a sample IVD test is received during the second step of measuring a subsequent calibration solution in a series and/or the subsequent calibration solution in a pair, interrupting the calibration procedure comprises completing the second step of measuring the subsequent calibration solution and aborting any following steps of the calibration procedure in order to execute the sample IVD test.

According to an embodiment, the method comprises executing a washing step with the first calibration solution before executing the sample IVD test. By doing so, the same conditions before sample measurement can be assured, while at the same time providing the opportunity to once again measure the first calibration solution in order to have the most up-to-date test-specific calibration parameters that can be determined by measuring the first calibration solution.

According to an embodiment, the method comprises updating at least part of the test-specific calibration parameters following the measurement of the first calibration solution or completed measurement of a pair of calibration solutions. By doing so, although the calibration procedure may not yet be completed and/or may still be interrupted, it can be assured that as soon as any test-specific calibration parameters can be determined based on the already measured calibration solution(s), these are also updated so that they may already be used in evaluating the next sample IVD test result if the calibration procedure is interrupted.

According to an embodiment, after executing the sample IVD test, following an interruption of the calibration procedure, the method comprises restarting the calibration procedure for calibration procedures using a single pair of calibration solutions and resuming the interrupted calibration procedure starting from the pair of calibration solutions the measurement of which was interrupted or aborted for calibration procedures using multiple pairs of calibration solutions. Thus, if the measurement of one or more pairs of calibration solution in a series comprising multiple calibration solution had already been completed, before interrupting the calibration procedure, there is no need to repeat the measurement for that or those pairs, and the test-specific calibration parameters can be updated as they are being determined.

According to an embodiment, the method comprises combining different calibration procedures into a single calibration cycle if they share the same or similar calibration validity periods and/or if the respective validity periods have the same expiration, by adding up the respective calibration solutions and/or pairs of calibration solutions to the same series.

The term "sample" refers to a biological material potentially containing one or more analytes of interest and whose detection or analysis, qualitative and/or quantitative measurement, may be associated to a clinical condition. The sample can be derived from any biological source, such as a physiological fluid, including blood, saliva, ocular lens fluid, cerebral spinal fluid, sweat, urine, milk, ascites fluid, mucous, synovial fluid, peritoneal fluid, amniotic fluid, tissue, cells or the like. The test sample can be pretreated prior to use, such as preparing plasma or serum from blood, diluting viscous fluids, lysis or the like; methods of treatment can involve filtration, centrifugation, distillation, concentration, inactivation of interfering components, and the addition of reagents. A sample may be used directly as obtained from the source in some cases or following a pretreatment and/or sample preparation workflow to modify the character of the sample, e.g. after adding an internal standard, after being diluted with another solution or after having being mixed with reagents e.g. to enable carrying out one or more in vitro diagnostic tests, or for enriching (extracting/separating/concentrating) analytes of interest and/or for removing matrix components potentially interfering with the detection of the analyte(s) of interest.

According to one aspect, the sample is blood or a blood derivative such as plasma or serum.

According to certain aspects, analytes of interest are hemoglobin, hemoglobin derivatives such as deoxygenated Hemoglobin (HHb), Oxyemoglobin (O₂Hb), CarB-oxyhemoglobin (COHb), Methemoglobin (MetHb), bilirubin, Urea, Creatinine, typically measured in the optical detection unit. In turn, additional sample parameters can be deduced based on the previous measurements such as Oxygen saturation (SO₂ = O₂Hb / (O₂Hb + HHb)) and total Hemoglobin (tHb = sum of all Hemoglobins). Other analytes of interest are gases, such as pO₂ and pCO₂, blood electrolytes such as Sodium (Na⁺), Potassium (K⁺), Chloride (Cl⁻), Calcium (Ca⁺⁺), protons (H⁺) in relation to pH, metabolites such as Glucose and Lactate, and the like, typically measured in a flow-through sensor path. The list is however not exhaustive.

An in-vitro diagnostic (IVD) system for carrying out IVD tests on patient samples is herein also disclosed, the IVD system comprising at least one measurement unit, a fluidic system for transporting at least calibration solutions and samples to the at least one measurement unit and a controller running a computer-readable program provided with instructions to perform operations associated with the method of managing calibration according to any of the disclosed embodiments.

The term "in-vitro diagnostic system" as used herein refers to an automated or semi-automated analytical apparatus configured to analyze samples in vitro in order to provide information for screening, diagnosis or treatment monitoring purposes. The IVD system can be designed and configured according to the medical area of application, the parameters to be determined and corresponding laboratory workflows. For example, in a point-of-care testing environment, IVD systems can vary from handheld devices with low throughput, short turn-around time and limited number of measurable parameters to compact benchtop instruments with higher throughput and higher number of measureable parameters. Such IVD systems are designed to detect certain types of parameters, e.g. gases, electrolytes, metabolites, clinical chemistry analytes, immunochemistry analytes, coagulation parameters, hematology parameters, etc. Depending on the parameters of interest, a variety of different sample IVD tests, including different analytical methods and different detection technologies can be applied. For example, in the field of blood gas and electrolyte testing, electrochemical measuring principles and/or conductivity measuring principles and/or optical detection methods are typically used. An IVD system typically comprises a plurality of functional units, each dedicated to a specific task and cooperating with each other in order to enable automated sample processing and analysis. Such functional units may include e.g. a sample input interface for receiving a sample, a fluidic system, at least one measurement unit or detection unit, a fluid-supply unit, and the like. One or more functional units may be integrated into a larger unit or module in order to simplify the operation of the IVD system.

According to an embodiment, the at least one measurement unit is an optical detection unit comprising a cuvette arranged between a light source and a photodetector, enabling measuring the intensity of light in a part of the spectrum, that is transmitted or emitted by particular substances, such as analytes or calibration materials present in a sample or calibration solution placed therein. The optical detection unit may be embodied as a flow-through optical detection unit comprising a flow-through cuvette or flow-through optical path enabling fluids such as samples and calibration solutions and possibly other fluids, including e.g. air, to flow in and out.

According to an embodiment the IVD system may alternatively or additionally comprise a "flow-through sensor path" as measuring unit, that is a fluidic conduit comprising one or more sensors that a sample or calibration solution flowing through the sensor path can come in contact with, e.g. arranged sequentially along the path, e.g. a sensor for each different parameter/analyte to be detected, and may be embodied in a replaceable cartridge-like structure comprising a plurality of sensors, possibly distributed across a plurality of sensor paths. In alternative, the IVD system may comprise a plurality of measurement units, each having a sensor path comprising a sensor dedicated to one parameter/analyte, and which may also be replaceable or not. A sample or calibration solution may thus flow into the one or more sensor paths and different parameters/analytes may be determined by respective sensors. The term "sensor" is herein generically used to indicate a detector configured to detect sample parameters by generating a correlated signal output that can be quantified and digitized. The sensor can be e.g. a biosensor, a chemical sensor or a physical sensor. The sensor can be selective or specific with respect to one sample parameter of interest or can be configured to detect and quantify a plurality of different sample parameters of interest. Depending on the type of sensor, a sensor can comprise a plurality of sensory elements. The term "sensory element" therefore refers to a part of a sensor (e.g. to a working electrode, a reference electrode, a counter electrode) that in combination with one or more other sensory elements forms a fully functional sensor. According to an embodiment, the flow-through sensor path comprises any one or more of a pO₂ sensor, a pCO₂ sensor, a pH sensor, one or more ion selective electrode (ISE) sensors for determining electrolyte values such Na⁺, K⁺, Ca²⁺ and Cl⁻, one or more metabolite sensors for determining parameters such as lactate and glucose. The sensors may be e.g. respectively based on the amperometric, potentiometric or conductometric principle.

The IVD system may further comprise at least one pump, e.g. a peristaltic pump, syringe pump, membrane pump or any other suitable pump, for transporting fluids, including samples and calibration solutions through the fluidic system.

Since the measurement unit and at least part of the fluidic system are the same as the ones used for transporting and measuring the calibration solutions, this is the reason why a sample IVD test cannot be executed while a calibration procedure is being executed.

The IVD system may comprise a dedicated sample input interface for introducing samples into the IVD system. The sample input interface may be arranged at a position conveniently accessible by an operator and configured to transfer a sample from a sample container, brought up by the operator, into the in-vitro diagnostic system. It may e.g. comprise a sample input port comprising an outer input-port side configured for coupling, attaching, connecting, sitting, introducing or plugging-in a sample container, e.g. of the capillary-type or syringe-type, and an inner input-port side coupled to or for coupling to one end of a sample-input conduit, the sample input conduit being fluidically connected or connectable to the measurement unit. The IVD system may comprise a fluid supply unit such as a module or component of the IVD system comprising one or more fluid reservoirs comprising the calibration solutions, among possible other fluids such as quality control (QC) samples, and possibly also one or more waste containers where fluids circulated through the fluidic system may be wasted at the end of the process.

The term "controller" encompasses any physical or virtual processing device and in particular a programmable logic computer with a processor running a computer-readable program provided with instructions to perform operations associated with the method of managing calibration according to any of the disclosed embodiments. The controller may be integrated into the in-vitro diagnostic system or be a separate logic entity in communication with the in-vitro diagnostic system. In some embodiments, the controller might be integral with a data management unit, may be comprised by a server computer and/or be distributed/shared across/between a plurality of in-vitro diagnostic systems. The controller may be also configurable to control the in-vitro diagnostic system in a way that workflow(s) and workflow step(s) are conducted by the in-vitro diagnostic system. In particular, the controller may communicate and/or cooperate with a scheduler and/or data manager and/or user input interface and/or sample input interface in order to take into account incoming sample IVD test orders and/or received sample IVD test orders, and a number of scheduled process operations associated with the execution of the sample IVD test orders in order to plan when to start and when to interrupt execution of a calibration procedure, and when to restart or resume a calibration procedure. In particular, the controller may be configured to execute any of the method steps according to any of the above described embodiments.

Other and further objects, features and advantages will appear from the following description of exemplary embodiments and accompanying drawings, which serve to explain the principles more in detail.

### Brief description of the drawings

FIG. 1 shows a table with an arbitrary selection of generic calibration solutions used in calibration procedures related to some sample IVD tests.
FIG. 2 shows examples of calibration cycles comprising executing calibration procedures in order to determine test-specific calibration parameters by using different series of calibration solutions selected from those in FIG. 1.
FIG. 3 shows a schematic example of executing a calibration cycle where the repetition of the calibration procedure has been completed and therefore it is possible to update all test-specific calibration parameters, used for evaluating glucose test results in this example.
FIG. 4 is a variant of FIG. 3 where repetition of the calibration procedure (solid line) has been interrupted, and therefore it is possible to only partially update the glucose-specific calibration parameters.
FIG. 5 shows another schematic example of executing a calibration cycle where the repetition of the calibration procedure has been completed and therefore it is possible to update all test-specific calibration parameters, used for evaluating Ca⁺⁺ test results in this example.
FIG. 6 is a variant of FIG. 5 where repetition of the calibration procedure has been interrupted, and therefore it is possible to only partially update the Ca⁺⁺-specific calibration parameters.
FIG. 7 schematically shows sub-steps during execution of a calibration procedure using a series of calibration solutions.
FIG. 8 schematically shows a more comprehensive computer-implemented method of automatically managing calibration of an IVD system, including aspects of FIG. 1 to 7.
FIG. 9 shows schematically an IVD system for carrying out sample IVD tests comprising a controller configured to execute the method of managing calibration according to FIG. 8.

Skilled artisans appreciate that elements in the figures are illustrated for simplicity and clarity and have not necessarily been drawn to scale. For example, the dimensions of some of the elements in the figures may be exaggerated relative to other elements whereas other elements may have been left out or represented in a reduced number in order to enhance clarity and improve understanding of the aspects of the present disclosure.

### Detailed description

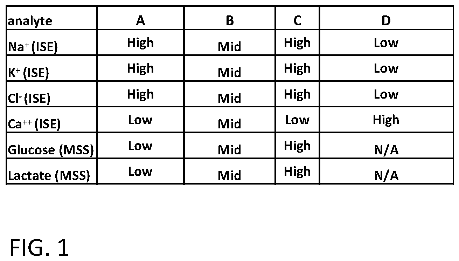

FIG. 1 shows a table with an arbitrary selection of generic calibration solutions comprising known values of calibration materials or standards corresponding to different levels, generally indicated as "high", "mid", "low", of analytes of interest possibly present in a sample, and that can be used in one or more calibration procedures in order to determine test-specific calibration parameters, used to evaluate the sample IVD test results, e.g. to determine the presence and quantity of the respective analytes in a sample. In this example, the analytes or sample parameters of interest related to respective sample IVD tests are electrolytes such as Na⁺, K⁺, Cl⁻ and Ca⁺⁺ that can be detected by Ion Selective Electrodes (ISE) and metabolites such as glucose and lactate that can be detected by respective metabolite sensors (MSS). The calibration solutions may alternatively or additionally comprise other calibration materials or standard related to other analytes or sample parameters of interest and less or more calibration solutions may be used depending on the particular sample IVD test(s), respective measuring principle and IVD system, including e.g. dynamic range, sensitivity, etc... No specific values are therefore given in this example.

ISE sensors typically work according to the potentiometric measuring principle. They differ only by different membrane materials that enable sensitivity for the respective electrolytes.

The glucose sensor typically makes use of glucose oxidase enzyme, by which glucose is oxidized into gluconolactone with oxygen from the air. The H₂O₂ that is formed in this process is determined amperometrically by a manganese dioxide/carbon electrode. Since the sensor restores the oxygen that is needed for oxidation of glucose in the enzymatic reaction, the glucose value is measured independently of the oxygen concentration in the test sample.

The lactate sensor typically makes use of lactate oxidase enzyme, by which lactate is oxidized into pyruvate with oxygen from the air. The H₂O₂ that is formed in this process is determined amperometrically in a manner similar to the glucose sensor.

Calibration solution A listed in the table is typically used and measured more frequently than the other calibration solutions, e.g. always as first calibration solution and/or between different calibration solutions and may be used also as wash solution, e.g. before and/or after introducing a sample.

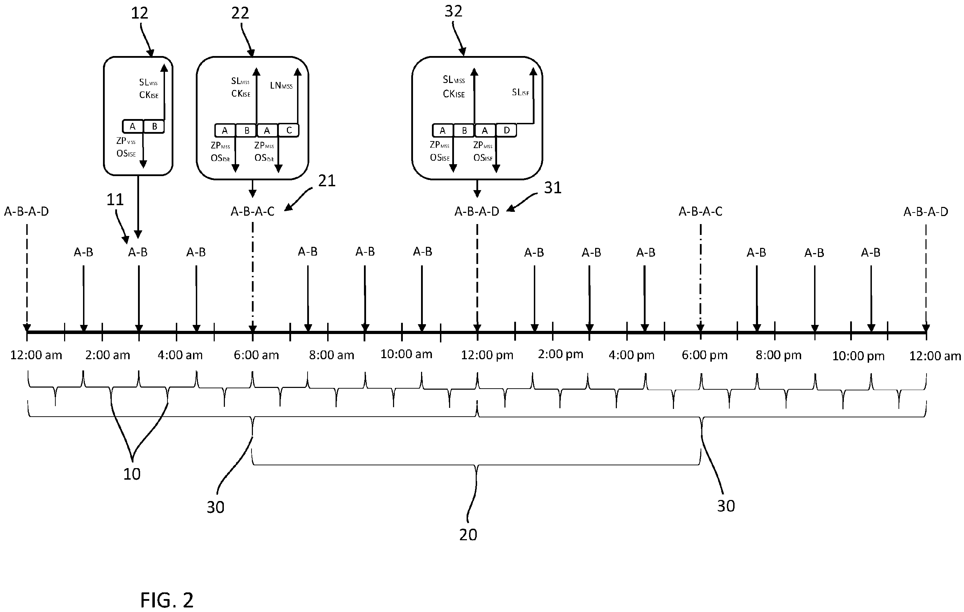

FIG. 2 shows examples of calibration cycles 10, 20, 30, comprising executing calibration procedures 11, 21, 31 respectively in order to determine test-specific calibration parameters 12, 22, 32 respectively used in evaluating sample IVD test results, and repeating the calibration procedures 11, 21, 31 in order to update the test-specific calibration parameters 12, 22, 32 before expiry of a respective calibration validity period, i.e. before starting new cycles 10, 20, 30, wherein different calibrations procedures 11, 21, 31 have different calibration validity periods respectively, and wherein executing different calibration procedures 11, 21, 31/cycles 10, 20, 30 comprises using different series of calibration solutions selected e.g. from those of FIG. 1, respectively, a series comprising at least two different calibration solutions, e.g. 2, 4 and possibly more, e.g. 6 (not shown) or more. In particular, in this example, a series of calibration solutions comprises a single pair A-B or multiple pairs A-B-A-C, A-B-A-D of calibration solutions A, B, C, D, each pair A-B, A-C, A-D comprising a common calibration solution A as first calibration solution and any other different calibration solution B, C, D as second or subsequent calibration solution. Other combinations (not shown) are possible, such as A-C, A-D, A-B-A-C-A-D and so on, and in general any combination with any number of calibration solutions is possible, not necessarily in pairs, depending on the particular sample IVD test(s) and content of calibration solutions.

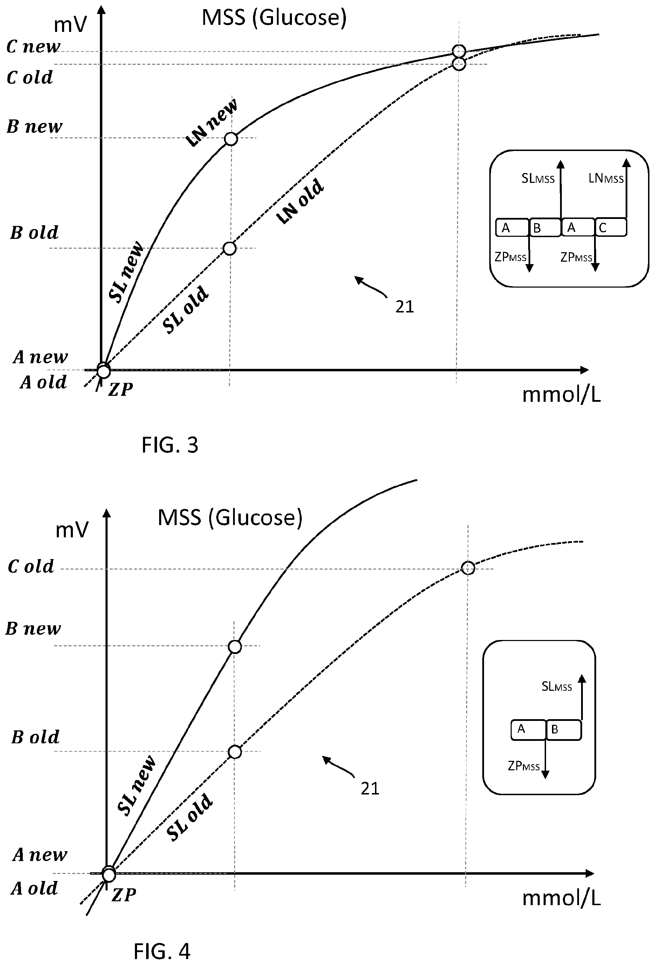

Depending on the particular calibration procedure 11, 21, 31 and respective series of calibration solutions used A-B, A-B-A-C, A-B-A-D respective test-specific calibration parameters 12, 22, 32 can be determined. Exemplary test-specific calibration parameters 12, 22, 32 that can be determined with reference to the analytes or sample parameters of interest as e.g. indicated in FIG. 1 and with respect to a respective calibration line or curve, include the slope (SL), the offset (OS), the linearity (LN), the zero point (ZP), explained in more detail in FIG. 3-6 by means of examples. Each calibration parameter is indicated together with a subscript next to it that refers to respective analytes or sample parameters of interest. The electrolytes Na⁺, K⁺, Cl⁻ and Ca⁺⁺ are generally indicated under the same group ISE, for simplicity. The metabolites glucose and lactate are generally indicated under the same group MSS respectively. The abbreviation CK (Check) can refer either to the calibration parameter OS or SL if not specified. It can be also noted that after each calibration solution that is used, a subset of the test-specific calibration parameters 12, 22, 32 may be determined.

In this example, executing the cycle 10 comprises executing the calibration procedure 11, comprising using the calibration solutions A-B every 1.5 hours, meaning that the calibration validity period for the calibration procedure 11 is 1.5 hours and that a repetition of the calibration procedure has to be executed before expiration of this calibration validity period. Executing the calibration cycle 20 comprises executing the calibration procedure 21, comprising using the calibration solution C every 12 hours, meaning that the calibration validity period for the calibration procedure 21 is 12 hours and that a repetition of the calibration procedure has to be executed before expiration of this calibration validity period. In particular, the calibration procedure 21, includes using the pair of calibration solutions A-C combined with the pair of calibration solutions A-B of the calibration procedure 11 into a single calibration cycle 20. This is an example of two calibration procedures 11, 21 having different calibration validity periods 1.5 hours and 12 hours respectively that expire however at the same time and are therefore combined by adding up the respective calibration solutions and/or pairs of calibration solutions to the same series. Analogously, executing the calibration cycle 30 comprises executing the calibration procedure 31, comprising using the calibration solution D every 12 hours, meaning that the calibration validity period for the calibration procedure 31 is 12 hours and that a repetition of the calibration procedure has to be executed before expiration of this calibration validity period. In particular, the calibration procedure 31, includes using the pair of calibration solutions A-D combined with the pair of calibration solutions A-B of the calibration procedure 11 into a single calibration cycle 30. This is another example of two calibration procedures 11, 21 having different calibration validity periods 1.5 hours and 12 hours respectively that expire however at the same time and are therefore combined by adding up the respective calibration solutions and/or pairs of calibration solutions to the same series. On the other hand, the calibration procedures 21 and 31 both have calibration validity period of 12 hours but they expire at different times respectively since they are staggered with a period of 6 hours in between.

FIG. 3 and FIG. 4 taken together show a specific example of calculating the result of a calibration procedure that is the process of constructing a line or a curve, or a mathematical function, that has the best fit to the measured calibration points. In this case, the calibration curve is that used for evaluating glucose test results that can be determined by measuring the calibration solutions A, B and C executing the calibration procedure 21 in FIG. 2. This is because the glucose calibration curve is a non-linear curve requiring using three levels of calibration solutions. The dashed curves in FIG. 3 and 4 refer to a completed calibration procedure using all three levels of calibration solutions A old, B old and C old, at the beginning of a cycle 20 in FIG. 2, by which the glucose-test-specific calibration parameters ZP, SL old and LN old have been determined and need to be updated by repetition of the calibration procedure 21, before expiration of the calibration validity period, in order to determine the new glucose-test-specific calibration parameters ZP, SL new and LN new and to update the old glucose-test-specific calibration parameters ZP, SL old and LN old. The difference between FIG. 3 and FIG. 4 is that in FIG. 3 the repetition of the calibration procedure 21 (solid line curve) has been completed and therefore it is possible to determine all three new glucose-test-specific calibration parameters ZP, SL new and LN new, whereas in FIG. 4 repetition of the calibration procedure 21 (solid line curve) has been interrupted after measuring A new and B new, hence it is not possible to determine the linearity (LN new) of the new curve without having measured C new yet. It is however possible to determine, at least temporary, ZP and SL new, thereby updating at least part of the glucose-test-specific calibration parameters, until the calibration procedure is restarted or resumed. For the time being LN old continues to be used.

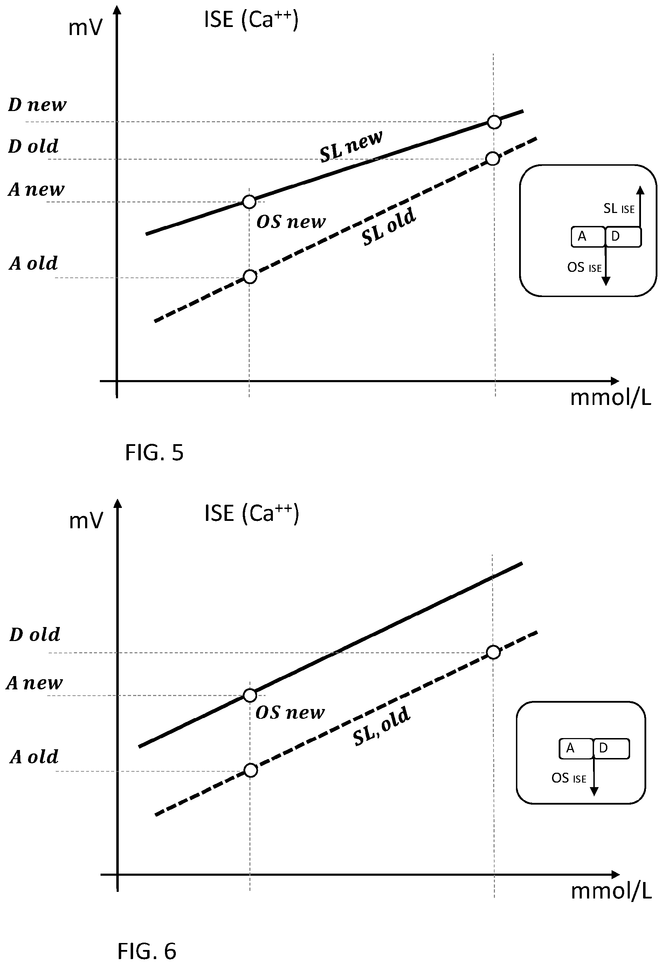

FIG. 5 and FIG. 6 taken together show another specific example of calculating the result of a calibration procedure. In this case, the calibration curve is that used for evaluating Ca⁺⁺ test results that can be determined by measuring the calibration solutions A and D executing the calibration procedure 31 in FIG. 2. This is because the Ca⁺⁺ calibration curve is linear and therefore using two levels of calibration solutions is sufficient. The dashed lines in FIG. 5 and 6 refer to a completed calibration procedure using both levels of calibration solutions A old and D old, at the beginning of a cycle 30 in FIG. 2, by which the Ca⁺⁺-test-specific calibration parameter OS old (not shown) with respect to a previous cycle 30 and SL old have been determined and need to be updated by repetition of the calibration procedure 31, before expiration of the calibration validity period, in order to determine the new Ca⁺⁺-test-specific calibration parameters OS new and SL new and to update the old Ca⁺⁺-test-specific calibration parameters OS old (not shown) and SL old. The difference between FIG. 5 and FIG. 6 is that in FIG. 5 the repetition of the calibration procedure 31 (solid line) has been completed and therefore it is possible to determine both new Ca⁺⁺-test-specific calibration parameters OS new and SL new, whereas in FIG. 6 repetition of the calibration procedure 31 (solid line) has been interrupted after measuring A new, hence it is not possible to determine the new slope (SL new) of the new line without having measured D new yet. It is however possible to determine, at least temporary OS new, thereby updating at least part of the Ca⁺⁺-test-specific calibration parameters, until the calibration procedure is restarted or resumed. For the time being SL old continues to be used.

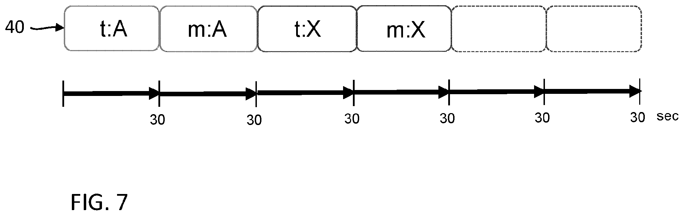

FIG. 7 shows a further aspect about executing a calibration procedure using a series 40 of calibration solutions, a series 40 comprising at least two different calibration solutions A, X, where X can be any calibration solution, e.g. B, C, D, where using a calibration solution A, X, comprises a first step of transporting t:A, t:X the calibration solution A, X respectively to a measurement unit and a second step of measuring m:A, m:X the calibration solution A, X respectively by the measurement unit. In particular each step t:A, t:X, m:A, m:X has the same time duration, e.g. 30 seconds in this example, meaning that transporting and measuring a calibration solution takes up 60 seconds. This is only an example, which can be adapted according to the particular IVD system and calibration solutions used.

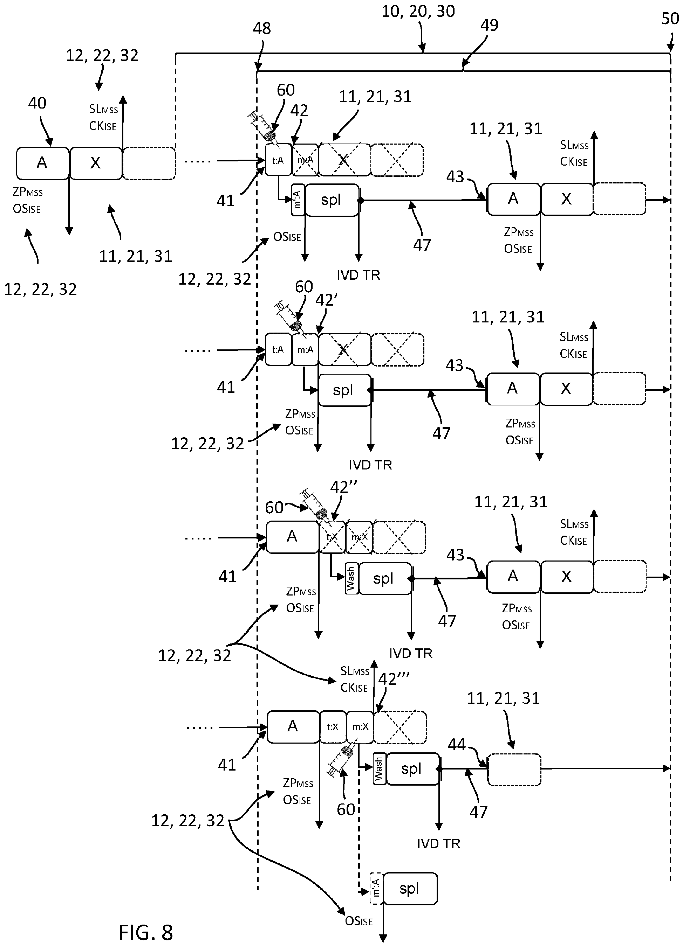

FIG. 8 schematically shows a more comprehensive computer-implemented method of automatically managing calibration of an IVD system, the method comprising executing calibration cycles 10, 20, 30, a calibration cycle 10, 20, 30 comprising executing a calibration procedure 11, 21, 31 in order to determine test-specific calibration parameters 12, 22, 32 used in evaluating sample IVD test results (IVD TR), and repeating the calibration procedure in order to update the test-specific calibration parameters 12, 22, 32 before expiry 50 of a respective calibration validity period, where different calibrations procedures 11, 21, 31 / cycles 10, 20, 30 possibly have different calibration validity periods respectively, and where executing different calibration procedures 11, 21, 31 / cycles 10, 20, 30 comprises using different series 40 of calibration solutions A, X, a series 40 comprising at least two different calibration solutions A, X and where using a calibration solution A, X comprises a first step of transporting t:A, t:X the calibration solution to a measurement unit and a second step of measuring m:A, m:X the calibration solution by the measurement unit, as also shown in FIG. 7. In particular, for each calibration cycle 10, 20, 30 the method comprises starting 41 repetition of the calibration procedure 11, 21, 31 at the earliest at a start 48 of a buffer time period 49 before expiry 50 of the respective calibration validity period, interrupting 42, 42', 42", 42‴ the calibration procedure 11, 21, 31 upon receiving an order 60 to execute a sample IVD test (spl) and restarting 43 or resuming 44 the calibration procedure 11, 21, 31 after executing the sample IVD test (spl), as long as repetition of the calibration procedure 11, 21, 31 can be restarted 43 or resumed 44 within the buffer time period 49. In addition, interrupting 42, 42', 42", 42‴ the calibration procedure 11, 21, 31 comprises different steps depending on whether the order 60 to execute a sample IVD test (spl) is received during using a first calibration solution A or a subsequent calibration solution X in a series 40 of calibration solutions A, X, and whether it is received during the first step of transporting t:A, t:X the first or a subsequent calibration solution A, X respectively to the measurement unit or the second step of measuring m:A, m:X the first or a subsequent calibration solution A, X respectively.

According to an embodiment, when the order 60 to execute a sample IVD test (spl) is received during the first step t:A of transporting the first calibration solution A in a series 40 and/or the first calibration solution A in a pair A, X to the measurement unit, interrupting 42 the calibration procedure 11, 21, 31 comprises completing the first step t:A of transporting the first calibration solution A to the measurement unit and executing a measurement step m':A of the first calibration solution A with a reduced measurement time and aborting any following steps (marked with a dashed cross) of the calibration procedure 11, 21, 31 in order to execute the sample IVD test (spl).

According to an embodiment, when the order 60 to execute a sample IVD test (spl) is received during the second step m:A of measuring the first calibration solution A in a series 40 and/or the first calibration solution A in a pair A, X, interrupting 42' the calibration procedure comprises completing the second step m:A of measuring the first calibration solution A and aborting any following steps (marked with a dashed cross) of the calibration procedure in order to execute the sample IVD test (spl).

According to an embodiment, when the order 60 to execute a sample IVD test (spl) is received during the first step t:X of transporting a subsequent calibration solution X in a series 40 and/or the subsequent calibration solution X in a pair A, X to the measurement unit, interrupting 42" the calibration procedure 11, 21, 31 comprises aborting the first step t:X of transporting the subsequent calibration solution X to the measurement unit and any following steps (marked with a dashed cross) of the calibration procedure 11, 21, 31 in order to execute the sample IVD test (spl). The method further comprises executing a washing step (wash) before executing the sample IVD test (spl), the washing step (wash) having a duration shorter than any interrupted step. Particularly the method may comprise executing the washing step (wash) with the first calibration solution A before executing the sample IVD test (spl). The method may further comprise executing a measurement step m':A of the first calibration solution A with a reduced measurement time before executing the sample IVD test (spl).

According to an embodiment, when the order 60 to execute a sample IVD test (spl) is received during the second step m:X of measuring a subsequent calibration solution X in a series 40 and/or the subsequent calibration solution X in a pair A, X, interrupting 42‴ the calibration procedure 11, 21, 31 comprises completing the second step m:X of measuring the subsequent calibration solution X and aborting any following steps (marked with a dashed cross) of the calibration procedure 11, 21, 31 in order to execute the sample IVD test (spl). The method further comprises executing a washing step (wash) before executing the sample IVD test (spl), the washing step (wash) having a duration shorter than any interrupted step. Particularly, the method may comprise executing the washing step (wash) with the first calibration solution A before executing the sample IVD test (spl). The method may further comprise executing a measurement step m':A of the first calibration solution A with a reduced measurement time before executing the sample IVD test (spl).

With continued reference to FIG. 8, the method further comprises updating at least part of the test-specific calibration parameters 12, 22, 32 following the measurement of the first calibration solution A or completed measurement of a pair of calibration solutions A, X.

Also, after executing the sample IVD test (spl), the method comprises restarting 43 the calibration procedure 11, 21, 31 for calibration procedures using a single pair of calibration solutions A, X or interrupted 42, 42', 42" during using the first pair of calibration solution A, X and resuming 44 the interrupted calibration procedure 11, 21, 31 starting from the pair of calibration solutions the measurement of which was interrupted or aborted 42‴ for calibration procedures using multiple pairs of calibration solutions.

In addition, restarting 43 or resuming 44 the calibration procedure 11, 21, 31 comprises waiting a predefined time 47 after executing the sample IVD test (spl) as long as repetition of the calibration procedure can be restarted 43 or resumed 44 within the buffer time period 49.

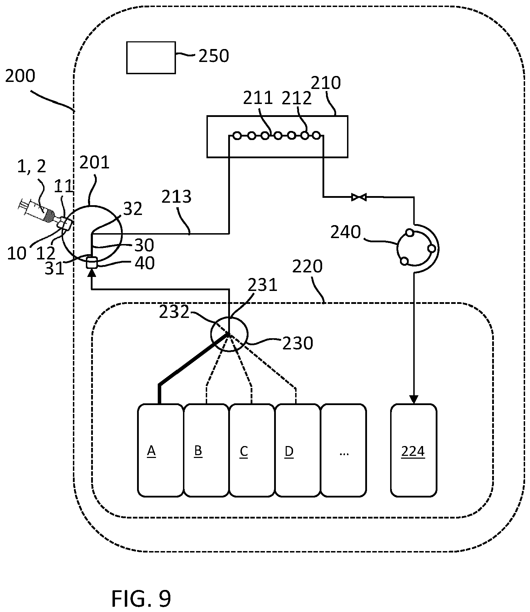

FIG. 9 shows schematically an IVD system 200 for carrying out sample IVD tests comprising at least one measurement unit 210, a fluidic system 213 for transporting at least calibration solutions A, B, C, D and samples 2 to the at least one measurement unit 210 and a controller 250 running a computer-readable program provided with instructions to perform operations associated with the method of managing calibration according to any of the above described embodiments.

The measurement unit 210 comprises a flow-through sensor path 211 comprising a plurality of sensors 212, such as ISE sensors and metabolite sensors. The IVD system 200 further comprises a pump 240, such as a peristaltic pump, located downstream of the of the fluidic system 213, and a fluid-supply unit 220 comprising a plurality of fluids including the calibration solutions A, B, C, D, and a waste container 224 where fluids circulated through the fluidic system 213 may be disposed of, by the action of the pump 240. The IVD system 200 further comprises a fluid-selection valve 230 for selecting between the fluids A, B, C, D and/or air 232.

The IVD system 200 further comprises a sample input interface 201, comprising a sample input port 10 comprising an outer input-port side 11 configured for plugging-in an open end of a sample container 1 and an inner input-port side 12. The sample input interface 201 further comprises an aspiration needle 30 comprising an upstream end 31 and a downstream end 32. The downstream end 32 of the aspiration needle 30 is fluidically connected to the fluidic system 213 whereas the upstream end 31 is configured to alternately couple to the inner input-port side 12 in order to aspirate a sample 2 from the sample container 1 plugged in the outer input-port side 11 and to a fluid-supply unit port 40 fluidically connected to a common outlet port 231 of the fluid-selection valve 230. The fluidic system 213 may be however directly connected to the outlet port 231 of the fluid-selection valve 230, whereas samples may be introduced via a different fluidic line separately connected to the fluid-selection valve 230, for example.

Modifications and variations of the disclosed aspects are also certainly possible in light of the above description. It is therefore to be understood, that within the scope of the appended claims, the invention may be practiced otherwise than as specifically devised in the above examples.

Particularly, it is to be understood that at least some of the drawings or parts are only schematic and provided as way of example only. Also the relationship between elements may be other than the one shown, whereas parts not relevant for the purpose of this disclosure have been omitted.

Also, reference throughout the preceding specification to "one aspect", "an aspect", "one example" or "an example", "one embodiment" or "an embodiment" means that a particular feature, structure or characteristic described in connection with the aspect or example or embodiment is included in at least one aspect, example or embodiment. Thus, appearances of the phrases "in one aspect", "in one aspect", "one example" or "an example", "one embodiment" or "an embodiment", in various places throughout this specification are not necessarily all referring to the same aspect or example or embodiment.

Furthermore, the particular features, structures, or characteristics may be combined in any suitable combinations and / or sub-combinations in one or more aspects or examples or embodiments.

## Claims

1. A computer-implemented method of automatically managing calibration of an in-vitro diagnostic (IVD) system (200), the method comprising
executing calibration cycles (10, 20, 30), a calibration cycle (10, 20, 30) comprising executing a calibration procedure (11, 21, 31) in order to determine test-specific calibration parameters (12, 22, 32) used in evaluating sample IVD test results, and repeating the calibration procedure (11, 21, 31) in order to update the test-specific calibration parameters (12, 22, 32) before expiry (50) of a respective calibration validity period, wherein different calibrations procedures (11, 21, 31) / cycles (10, 20, 30) possibly have different calibration validity periods respectively, and wherein executing different calibration procedures (11, 21, 31) / cycles (10, 20, 30) comprises using different series (40) of calibration solutions (A, X) respectively, a series comprising at least two different calibration solutions (A, X), and wherein using a calibration solution (A, X) comprises a first step of transporting (t:A, t:X) the calibration solution (A, X) to a measurement unit (210) and a second step of measuring (m:A, m:X) the calibration solution (A, X) by the measurement unit (210),
wherein for each calibration cycle (10, 20, 30) the method comprises
- starting (41) repetition of the calibration procedure (11, 21, 31) at the earliest at a start (48) of a buffer time period (49) before expiry (50) of the respective calibration validity period,
- interrupting (42, 42', 42", 42‴) the calibration procedure (11, 21, 31) upon receiving an order (60) to execute a sample IVD test (spl) and restarting (43) or resuming (44) the calibration procedure (11, 21, 31) after executing the sample IVD test (spl), as long as repetition of the calibration procedure (11, 21, 31) can be restarted (43) or resumed (44) within the buffer time period (49),
wherein interrupting (42, 42', 42", 42‴) the calibration procedure (11, 21, 31) comprises different steps depending on whether the order (60) to execute a sample IVD test (spl) is received during using a first calibration solution (A) or a subsequent calibration solution (X) in a series (40) of calibration solutions (A, X) and whether it is received during the first step of transporting (t:A, t:X) the first or a subsequent calibration solution (A, X) to the measurement unit (210) or the second step of measuring (m:A, m:X) the first or a subsequent calibration solution (A, X).

2. The method according to claim 1 wherein, a series (40) of calibration solutions (A, X) comprises a single pair or multiple pairs of calibration solutions (A, X), each pair comprising a common calibration solution (A) as first calibration solution and any other different calibration solution (X, B, C, D) as the subsequent calibration solution.

3. The method according to claim 1 or 2 wherein, when the order (60) to execute a sample IVD test (spl) is received during the first step of transporting (t:A) the first calibration solution (A) in a series (40) and/or the first calibration solution (A) in a pair to the measurement unit (210), interrupting (42) the calibration procedure (11, 21, 31) comprises completing the first step of transporting (t:A) the first calibration solution (A) to the measurement unit (210) and executing a measurement step (m':A) of the first calibration solution (A) with a reduced measurement time and aborting any following steps of the calibration procedure (11, 21, 31) in order to execute the sample IVD test (spl).

4. The method according to claim 1 or 2 wherein, when the order (60) to execute a sample IVD test (spl) is received during the second step of measuring (m:A) the first calibration solution (A) in a series (40) and/or the first calibration solution (A) in a pair, interrupting (42') the calibration procedure (11, 21, 31) comprises completing the second step of measuring (m:A) the first calibration solution (A) and aborting any following steps of the calibration procedure (11, 21, 31) in order to execute the sample IVD test (spl).

5. The method according to claim 1 or 2 wherein, when the order (60) to execute a sample IVD test (spl) is received during the first step of transporting (t:X) a subsequent calibration solution (X) in a series (40) and/or the subsequent calibration solution (X) in a pair to the measurement unit (210), interrupting (42") the calibration procedure (11, 21, 31) comprises aborting the first step of transporting (t:X) the subsequent calibration solution (X) to the measurement unit (210) and any following steps of the calibration procedure (11, 21, 31) in order to execute the sample IVD test (spl).

6. The method according to claim 1 or 2 wherein, when the order (60) to execute a sample IVD test (spl) is received during the second step of measuring (m:X) a subsequent calibration solution (X) in a series (40) and/or the subsequent calibration solution (X) in a pair, interrupting (42‴) the calibration procedure (11, 21, 31) comprises completing the second step of measuring (m:X) the subsequent calibration solution (X) and aborting any following steps of the calibration procedure (11, 21, 31) in order to execute the sample IVD test (spl).

7. The method according to claim 5 or 6 comprising executing a washing step with the first calibration solution (A) before executing the sample IVD test (spl).

8. The method according to claim 7 comprising executing a measurement step (m':A) of the first calibration solution (A) with a reduced measurement time before executing the sample IVD test (spl).

9. The method according to any of the claims 3 to 8 comprising updating at least part of the test-specific calibration parameters (12, 22, 32) following the measurement of the first calibration solution (A) or completed measurement of a pair of calibration solutions (A, X).

10. The method according to any of the claims 2 to 9 wherein, after executing the sample IVD test (spl), the method comprises restarting (43) the calibration procedure (11, 21, 31) for calibration procedures (11) using a single pair of calibration solutions (A, X) or interrupted (42, 42', 42") while using the first pair of calibration solutions (A, X) and resuming (44) the interrupted (42‴) calibration procedure (11, 21, 31) starting from the pair of calibration solutions the measurement of which was interrupted or aborted for calibration procedures (21, 31) using multiple pairs of calibration solutions (A, X).

11. The method according to any of the preceding claims wherein restarting (43) or resuming (44) the calibration procedure (11, 21, 31) comprises waiting a predefined time (47) after executing the sample IVD test as long as repetition of the calibration procedure (11, 21, 31) can be restarted (43) or resumed (44) within the buffer time period (49).

12. The method according to any of the preceding claims comprising combining different calibration procedures (11, 21, 31) into a single calibration cycle (20, 30) if they share the same or similar calibration validity periods and/or if the respective validity periods have the same expiration, by adding up the respective calibration solutions and/or pairs of calibration solutions to the same series (40).

13. An in-vitro diagnostic (IVD) system (200) for carrying out sample IVD tests (spl) comprising at least one measurement unit (210), a fluidic system (213) for transporting at least calibration solutions (A, B, C, D) and samples (2) to the at least one measurement unit (210) and a controller (250) running a computer-readable program provided with instructions to perform operations associated with the method of managing calibration according to any of the claims 1 to 12.
